# FASCICULE DE BREVET EUROPEEN

(11) **EP 0 913 374 B1**
(45) Date de publication et mention de la délivrance du brevet: **30.01.2002**
(21) Numéro de dépôt: 98402636.9
(22) Date de dépôt: 23.10.1998
(51) Int. Cl.: C06B 25/34, C07D 487/22

(54) **Procédé d'obtention de la forme polymorphe epsilon de l'hexanitrohexaazaisowurtzitane**
Verfahren zur Herstellung der epsilon polymorphischen Form des Hexanitrohexaazaisowurtzitan
Process for obtaining the epsilon polymorphic form of hexanitrohexaazaisowurtzitane

(30) Priorité: 29.10.1997 FR 9713546
(43) Date de publication de la demande: 06.05.1999
(73) Titulaire: SOCIETE NATIONALE DES POUDRES ET EXPLOSIFS, F-75181 Paris Cédex 04 (FR)
(72) Inventeur: Bescond, Philippe, 91770 Saint Vrain (FR); Graindorge, Hervé, 91710 Vert le Petit (FR); Mace, Hélène, 91140 Villebon sur Yvette (FR)

(56) Documents cités:
- WO-A-97/20785
- US-A- 5 693 794
- DATABASE WPI Week 9506 Derwent Publications Ltd., London, GB; AN 95-041279 XP002069511 & JP 06 321962 A (ASAHI KASEI KOGYO K.K.) , 22 novembre 1995
- CHEMICAL ABSTRACTS, vol. 124, no. 24, 10 juin 1996 Columbus, Ohio, US; abstract no. 320923, XP002069506 & Y. HUANG ET AL.: PROC. BEIJING INT. SYMP. PYROTECH. EXPLOS., 3RD,1995, pages 167-169,
- CHEMICAL ABSTRACTS, vol. 121, no. 22, 28 novembre 1994 Columbus, Ohio, US; abstract no. 259139, XP002069507 & E. VON HOLTZ ET AL.: PROPELLANTS, EXPLOS., PYROTECH., vol. 19, no. 4, 1994, pages 206-212,
- CHEMICAL ABSTRACTS, vol. 120, no. 20, 16 mai 1994 Columbus, Ohio, US; abstract no. 248721, XP002069508 & F.M. FOLTZ ET AL.: PROPELLANTS, EXPLOS., PYROTECH., vol. 19, no. 1, 1994, pages 19-25,
- CHEMICAL ABSTRACTS, vol. 124, no. 22, 27 mai 1996 Columbus, Ohio, US; abstract no. 293607, XP002069509 & Z. FENG: BINGGONG XUEBAO, HUOHUANGONG FENCE, vol. 18, no. 1, 1996, pages 46-49,
- CHEMICAL ABSTRACTS, vol. 118, no. 14, 5 avril 1993 Columbus, Ohio, US; abstract no. 132609, XP002069510 & T.P. RUSSEL ET AL.: J. PHYS. CHEM., vol. 97, no. 9, 1993, pages 1993-1997,

## Description

La présente invention est relative à l'obtention de la forme polymorphe, appelée epsilon, de l'hexanitrohexaazaisowurtzitane.

L'invention se situe dans le domaine des poudres, propergols et explosifs, très couramment utilisés, notamment dans les industries d'armement.

Il existe, depuis quelques années, de nombreuses publications relatives au 2,4,6,8,10,12-hexanitro-2,4,6,8,10,12-hexaazatétracyclo (5.5.0.0^{5,9}.0^{3,11})dodécane, encore appelé hexanitrohexaazaisowurtzitane.

Ces publications décrivent les propriétés physiques, chimiques et détoniques de ce composé et/ou diverses formes polymorphes, ainsi que son utilisation dans des compositions explosives, des propergols ou des poudres pour armes.

On peut citer, par exemple, F. FOLTZ qui, dans Propellants, Explosives, Pyrotechnics 19, 63-69 (1994), étudie la stabilité thermique du polymorphe epsilon dans un poly(uréthanne-ester), et qui, dans Propellants, Explosives, Pyrotechnics 19, 19-25 (1994), étudie la stabilité thermique des quatre formes polymorphes appelées alpha, bêta, gamma et epsilon.

Toutefois, les informations concernant sa synthèse sont très rares, imprécises et insuffisantes pour que l'homme du métier, même avec ses connaissances générales, puisse la réaliser.

Si les auteurs des publications précitées mentionnent parfois que le composé a été obtenu à partir d'hexabenzylhexaazaisowurtzitane, ils ne décrivent jamais comment.

Les informations les plus précises concernant la synthèse figurent dans la demande de brevet PCT WO 97/20785 relative à la synthèse du tétraacétyldibenzylhexaazaisowurtzitane à partir de l'hexabenzylhexaazaisowurtzitane.

Il y est mentionné qu'on peut obtenir l'hexanitrohexaazaisowurtzitane à partir de ce composé intermédiaire acétylé en le faisant d'abord réagir avec un agent nitrosant puis ensuite avec un agent nitrant, mais aucun exemple d'une telle réaction n'est décrit, et aucune précision concernant les conditions opératoires (température, concentration des acides, milieu, etc...) n'est indiquée.

Arnold T. NIELSEN, au congrès de Long Beach (Californie, USA) organisé par l'American Defense Preparedness Association et tenu à l'Hotel Queen Mary les 27-29 octobre 1986, a également divulgué la synthèse du tétraacétyldibenzylhexaazaisowurtzitane, par réaction de l'hexabenzylhexaazaisowurtzitane avec l'anhydride acétique, en présence d'hydrogène et de Pd/C comme catalyseur.

L'auteur indique également avoir étudié de nombreuses conditions opératoires de nitration du tétraacétyldibenzylhexaazaisowurtzitane dans le but d'obtenir l'hexanitrohexaazaisowurtzitane, mais ce composé n'a jamais pu être obtenu.

Malgré ce préjugé et ces informations imprécises, des conditions opératoires ont maintenant été trouvées permettant l'obtention, avec un excellent rendement, de l'hexanitrohexaazaisowurtzitane à partir du tétraacétyldibenzylhexaazaisowurtzitane. L'hexanitrohexaazaisowurtzitane ainsi obtenu se présente sous la forme polymorphe alpha, en référence aux publications de FOLTZ précitées. Sa masse volumique est de 1,97 g/cm³.

Or, d'après ces mêmes publications de FOLTZ, c'est la forme polymorphe epsilon qui possède la masse volumique la plus élevée (2,04 g/cm³), et qui parait donc présenter le plus d'intérêt, notamment pour son utilisation dans les compositions pyrotechniques.

Si certaines propriétés et caractéristiques de la forme epsilon de l'hexanitrohexaazaisowurtzitane sont connues de l'homme du métier, les informations comprises dans ce même état de la technique, même complétées par les connaissances générales de l'homme du métier, ne permettent pas à celui-ci de la préparer et de l'isoler.

L'homme du métier est donc à la recherche de procédés permettant d'obtenir cette forme polymorphe epsilon.

Un premier procédé d'obtention de la forme polymorphe epsilon a été découvert consistant à tout d'abord mélanger de l'hexanitrohexaazaisowurtzitane de forme polymorphe quelconque, alpha par exemple, dans un prémélange comprenant :
- 20% à 40% en poids d'un polyazoture de glycidyle répondant à la formule générale 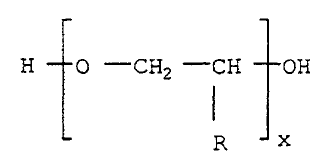 dans laquelle x est un nombre entier tel que 10 ≤ x ≤ 60 et R représente un groupement ( CH₂ )ₙ N₃ ou ―CH₂―CHN₃―CH₂N₃ dans lequel n est un nombre entier tel que 1 ≤ n ≤ 5,
- 60% à 80% en poids d'au moins un trinitrate d'un triol monomère comportant 3 à 12 atomes de carbone, puis à réaliser ensuite au moins un, de préférence plusieurs, et mieux encore au moins cinq, cycle thermique de chauffage du mélange à une température comprise entre 40°C et 60°C puis à une température comprise entre 10°C et 30°C, puis enfin à éliminer les constituants du prémélange par lavage avec un solvant organique.

Lorsqu'on mélange l'hexanitrohexaazaisowurtzitane et le prémélange, on obtient une suspension de l'hexanitrohexaazaisowurtzitane dans le prémélange, mais une faible partie est néanmoins dissoute.

De façon préférée, R représente ―CH₂N₃ et x est tel que 20 ≤ x ≤ 40.

De façon également préférée, le prémélange comprend 60% à 80% en poids d'un mélange de trinitrate de triméthyloléthane et de trinitrate de 1,2,4-butanetriol.

Selon une variante préférée, ce prémélange est constitué de 27,5% en poids de polyazoture de glycidyle, 35% en poids de trinitrate de triméthyloléthane, 35% en poids de trinitrate de 1,2,4-butanetriol, 1,25% en poids de 2-nitrodiphénylamine et 1,25% en poids de N-méthyl-paranitroaniline.

De façon générale, selon ce premier procédé, le rapport massique entre le prémélange et l'hexanitrohexaazaisowurtzitane à recristalliser, respectivement, est compris entre 10 et 100.

Un second procédé d'obtention de la forme polymorphe epsilon de l'hexanitrohexaazaisowurtzitane a été découvert consistant à réaliser une solution saturée d'hexanitrohexaazaisowurtzitane de forme polymorphe quelconque, alpha par exemple, dans un mélange acétone/toluène, puis à ensemencer cette solution saturée par quelques cristaux d'hexanitrohexaazaisowurtzitane de forme polymorphe epsilon, puis enfin à concentrer la solution par évaporation de l'acétone.

Ce second procédé est beaucoup plus simple à mettre en oeuvre, plus économique et plus facilement extrapolable au stade industriel que le premier, mais il présente l'inconvénient de ne pouvoir fournir que des produits de granulométrie relativement élevée, se présentant sous forme d'agglomérats, dont le diamètre médian est supérieur à 100µm, en général compris entre 100µm et 180µm, et ce, quelles que soient les conditions opératoires envisagées, notamment la taille des cristaux de semence et la vitesse d'évaporation de l'acétone.

Or, la formulation des matériaux énergétiques fortement chargés nécessite le plus souvent l'emploi, comme charge explosive pulvérulente, de plusieurs coupes granulométriques, en particulier dans la gamme de 10µm à 100µm, et, de préférence, des grains ayant des faces lisses et un faciès régulier, et non pas des agglomérats.

Le broyage du produit obtenu selon le second procédé précité est dangereux et économiquement très pénalisant.

L'homme du métier recherche donc un procédé permettant d'obtenir directement, sans broyage ultérieur, diverses coupes granulométriques de la forme epsilon de l'hexanitrohexaazaisowurtzitane de granulométrie bien définie, notamment des coupes granulométriques ayant un diamètre médian compris entre environ 10µm et environ 100µm avec, de préférence, des grains présentant des faces lisses et un faciès régulier.

La présente invention propose un tel procédé.

Il a été découvert, de façon inattendue, qu'on parvenait à de tels résultats en opérant selon les étapes réactionnelles suivantes :
- on réalise tout d'abord une solution saturée d'hexanitrohexaazaisowurtzitane de forme polymorphe quelconque, de préférence autre que la forme epsilon, par exemple alpha, dans un mélange comprenant d'une part un solvant organique de l'hexanitrohexaazaisowurtzitane choisi dans le groupe constitué par les esters, les nitriles, les éthers, les cétones autres que l'acétone, et leurs mélanges, et d'autre part un non solvant de l'hexanitrohexaazaisowurtzitane choisi dans le groupe constitué par les hydrocarbures aliphatiques, les hydrocarbures aromatiques et leurs mélanges, le solvant de l'hexanitrohexaazaisowurtzitane étant plus volatil (point d'ébullition inférieur) que le non solvant, et le solvant et le non solvant étant miscibles dans les proportions utilisées,
- on ensemence ensuite cette solution saturée par quelques cristaux d'hexanitrohexaazaisowurtzitane de forme polymorphe epsilon,
- puis on concentre la solution par évaporation, totale ou partielle, du solvant, ce qui provoque l'apparition de cristaux d'hexanitrohexaazaisowurtzitane de forme polymorphe epsilon qui demeurent en suspension dans le mélange enrichi en non solvant et que l'on peut récupérer ensuite par tout moyen usuel tel que la filtration.

Si la solution saturée contient de l'hexanitrohexaazaisowurtzitane en suspension, il est préférable d'éliminer cette suspension, par exemple par filtration, avant l'ensemencement, de façon à ne pas polluer le produit final obtenu.

Par ailleurs, de façon inattendue, on a constaté qu'on obtenait un produit de pureté plus élevée, c'est à dire exempt notamment d'autres formes polymorphes d'hexanitrohexaazaisowurtzitane, lorsque, lors de l'opération de concentration de la solution par évaporation du solvant organique, la température n'excède pas 50°C, c'est à dire lorsqu'elle est inférieure ou égale à 50°C, par exemple comprise entre 10°C et 50°C, de préférence comprise entre 20°C et 40°C.

Comme exemples de solvants organiques de l'hexanitrohexaazaisowurtzitane utilisables selon l'invention, on peut citer le formiate de méthyle, l'acétate de méthyle, l'acétate d'éthyle, l'acétate d'isopropyle, l'acétonitrile, les mélanges acétate d'éthyle-acétonitrile, le tétrahydrofurane (THF) et la méthyléthylcétone.

Comme exemples de non solvants de l'hexanitrohexaazaisowurtzitane, on peut citer le toluène, les xylènes, les alcanes tels que l'hexane, l'heptane et l'octane ainsi que les hydrocarbures aliphatiques halogénés, notamment les hydrocarbures aliphatiques chlorés tels que le 1,2-dichloroéthane.

Selon une variante préférée, le mélange solvant/non solvant comprend un solvant organique choisi dans le groupe constitué par les esters, de préférence dans le groupe constitué par les formiates et les acétates, et un non solvant choisi dans le groupe constitué par les alcanes, les hydrocarbures aliphatiques halogénés et les hydrocarbures aromatiques, de préférence dans le groupe constitué par les hydrocarbures aromatiques.

De façon particulièrement préférée, le solvant est choisi dans le groupe constitué par l'acétate de méthyle, l'acétate d'éthyle et l'acétate d'isopropyle, et le non solvant est choisi dans le groupe constitué par le toluène et les xylènes.

Le couple acétate d'éthyle/toluène est particulièrement préféré.

De façon générale, le rapport volumique solvant organique/non solvant respectivement, est compris entre 10/90 et 50/50, mieux encore entre 15/85 et 35/65.

Selon une autre variante préférée, pour réaliser la solution saturée d'hexanitrohexaazaisowurtzitane de forme polymorphe quelconque dans le mélange solvant organique/non solvant, on réalise d'abord une solution, saturée ou non saturée, d'hexanitrohexaazaisowurtzitane dans le solvant organique ou bien encore dans le solvant organique et une partie du non solvant et l'on ajoute ensuite le non solvant ou le reste du non solvant. Outre une simplification du procédé, on a constaté, de façon inattendue, qu'on obtenait ainsi des granulométries plus fines.

Selon une autre variante préférée, la quantité de semence représente entre 0,2% et 5% en poids de la quantité d'hexanitrohexaazaisowurtzitane de forme quelconque à recristalliser, de préférence entre 1% et 3% en poids.

Le procédé selon l'invention, simple à mettre en oeuvre, économique, facilement extrapolable au stade industriel, présente en plus l'avantage considérable de permettre l'obtention, en jouant sur des paramètres expérimentaux facilement contrôlables tels que la granulométrie de la semence, la méthode d'obtention des solutions saturées et la vitesse d'évaporation du solvant, de n'importe quelle coupe granulométrique recherchée dans la gamme 10µm-100µm, avec des grains ayant le plus souvent des faces lisses et un faciès régulier.

Si ce procédé nécessite de disposer au préalable, lorsqu'on le met en oeuvre pour la toute première fois, d'une faible quantité du produit recherché obtenu selon une autre méthode, la très faible proportion de semence nécessaire par rapport au produit à recristalliser rend ensuite le procédé parfaitement autonome.

Les exemples non limitatifs suivants illustrent l'invention et les avantages qu'elle procure.

Les exemples 1 à 4 concernent la préparation du produit de départ et de la semence nécessaires pour mettre en oeuvre le procédé selon l'invention.

L'exemple 4 est de plus un exemple comparatif permettant d'apprécier les avantages du procédé selon l'invention.

Les exemples 5 et suivants sont des exemples représentatifs du procédé selon l'invention.

### Exemple 1 : Synthèse du tétraacétyldibenzylhexaazaisowurtzitane.

Dans un réacteur double enveloppe de 250 ml muni d'une agitation magnétique, d'un réfrigérant à eau et d'une canne d'introduction équipée d'un fritté pour l'introduction d'hydrogène, on introduit, à la température ambiante (15-20°C), 67 ml de diméthylformamide (DMF), 17 ml d'anhydride acétique, 0,23 g (1,46 mmol) de bromobenzène, 20,8 g (29,4 mmol) d'hexabenzylhexaazaisowurtzitane et 1,15 g d'hydroxyde de palladium sur charbon (humidité : 50%, taux de palladium dans la matière sèche : 5%). Après purge de l'appareillage par un gaz inerte, et tout en introduisant de l'hydrogène dans le milieu et en maintenant sa pression dans le réacteur entre 1,13 10⁵Pa et 1,25 10⁵Pa, on porte le milieu réactionnel de la température ambiante jusqu'à 55°C en 3h, puis on maintient cette température pendant 2h.

On arrête l'introduction d'hydrogène, puis on introduit 158 ml d'acide acétique dans le milieu que l'on porte alors à une température comprise entre 80°C et 90°C.

On filtre ce milieu afin de séparer le catalyseur, puis on concentre le filtrat à 60°C-70°C sous une pression réduite de 2,5 10³Pa à 5 10³Pa (environ 20 mm à 40 mm Hg).

Après retour à la température ambiante, on reprend le résidu par 100 ml d'acétone. Le tétraacétyldibenzylhexaazaisowurtzitane obtenu, qui a précipité, est filtré et rincé par 50 ml d'acétone.

Après séchage 24h à 30°C sous une pression réduite de 5 10³Pa (environ 40 mm Hg), on obtient 12,1 g (rendement 80%) de tétraacétyldibenzylhexaazaisowurtzitane, identifié par conformité à des spectres de référence par spectrométrie de masse, infrarouge et RMN du proton à 60 MHz.

### Exemple 2 : Synthèse de l'hexanitrohexaazaisowurtzitane de forme polymorphe alpha.

Dans un réacteur d'un litre double enveloppe équipé d'une agitation mécanique et d'une sonde de température, on introduit, à 0°C, 313 g (3,37 mol) de N₂O₄ liquide.

On additionne, entre 0°C et 5°C, 133 g (0,259 mol) de tétraacétyldibenzylhexaazaisowurtzitane obtenu selon l'exemple 1.

On laisse remonter la température du milieu réactionnel jusqu'à 15-16°C (reflux du N₂O₄) puis on laisse le milieu sous agitation et au reflux du N₂O₄ pendant 20h.

Après avoir refroidi le milieu à 0°C, on ajoute, entre 0°C et 8°C, 667 ml d'un mélange sulfonitrique respectivement 20/80 en volumes, ce qui correspond à l'ajout de 12,8 mol d'acide nitrique.

On chauffe ensuite progressivement le milieu de façon à éliminer l'excès de N₂O₄ par distillation, puis, lorsque la température du milieu atteint 73-75°C, on laisse le milieu sous agitation durant 4h.

Après refroidissement à 40°C, on verse le milieu sur 2 1 d'un mélange eau-glace. Un solide décante que l'on récupère par filtration et lavage à l'eau chaude (40°C) sur filtre jusqu'à pH neutre des eaux de lavage.

Après séchage, on obtient 104 g d'hexanitrohexaazaisowurtzitane (rendement 97%) solide blanc identifié par RMN du proton à 200 MHz dans le diméthylsulfoxyde (DMSO), par RMN du carbone dans les mêmes conditions, par IR, par analyse élémentaire et par étude cristallographique aux rayons X.

Sa température de décomposition est voisine de 247°C et sa pureté peut être estimée supérieure à 95%.

Sa masse volumique est de 1,97 g/cm³ d'après les données cristallographiques obtenues par rayons X.

L'étude cristallographique d'un monocristal par rayons X montre que ce composé cristallise avec environ 25% molaires d'eau et qu'il présente une structure cristalline orthorhombique de groupe d'espace Pbca ayant les paramètres de mailles suivantes : a = 9,546Å, b = 13, 232Å, c = 23,634Å et Z= 8.

Par ailleurs, le spectre IR à transformée de Fourier d'une dispersion à 1% dans KBr présente, entre 700 cm⁻¹ et 1200 cm⁻¹, les pics caractéristiques de la forme polymorphe alpha, en référence à la publication de FOLTZ précitée, tableau 1 page 66. Les pics caractéristiques des formes epsilon, bêta et gamma ne sont pas observés.

L'hexanitrohexaazaisowurtzitane obtenu se trouve donc sous la forme polymorphe alpha.

### Exemple 3 : Obtention de l'hexanitrohexaazaisowurtzitane de forme polymorphe epsilon. Méthode par mélange.

Après mise en suspension avec dissolution partielle, à la température ambiante proche de 20°C, de 0,5 g d'hexanitrohexaazaisowurtzitane obtenu selon l'exemple 2 dans 15 g d'un prémélange constitué de :
- 27,5% en poids du polyazoture de glycidyle (PAG) de formule 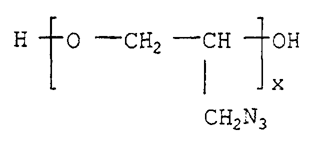
dans laquelle x est un nombre entier tel que 20 ≤ x ≤ 40 commercialisé par la SNPE sous la référence PAG-diol 1800 et dont la masse moléculaire moyenne en nombre est voisine de 1800,
- 35% en poids de trinitrate de triméthyloléthane,
- 35% en poids de trinitrate de 1,2,4-butanetriol,
- 1,25% en poids de 2-nitrodiphénylamine,
- 1,25% en poids de N-méthyl paranitroaniline,
on réalise six cycles thermiques successifs de 2h à 50°C, puis de 2h à 20°C. Chaque cycle est donc constitué d'une période de chauffage du mélange de 20°C à 50°C, d'une période de maintien de la température 2h à 50°C, d'une période de refroidissement du mélange de 50°C à 20°C, et d'une période de maintien de la température 2h à 20°C.

On élimine ensuite totalement les constituants du prémélange par lavage au chlorure de méthylène sur un filtre.

On recueille 0,47 g d'hexanitrohexaazaisowurtzitane, identifié comme selon l'exemple 2.

Sa masse volumique, à 20°C, est de 2,04 g/cm³ d'après les données cristallographiques obtenues par rayons X.

L'étude cristallographique d'un monocristal par rayons X montre que le produit présente une structure cristalline monoclinique de groupe d'espace P2₁/n, dont les paramètres de mailles sont : a = 8,864Å, b = 12,581Å, c = 13,388Å et Z = 4.

Par ailleurs, le spectre IR à transformée de Fourier d'une dispersion à 1% dans KBr présente, entre 700 cm⁻¹ et 1200 cm⁻¹, les pics caractéristiques de la forme polymorphe epsilon, en référence à la publication de FOLTZ précitée, tableau 1 page 66. Les pics caractéristiques des formes alpha, bêta et gamma ne sont pas observés.
L'hexanitrohexaazaisowurtzitane obtenu se trouve donc sous la forme polymorphe epsilon.

Par microscopie optique, on a constaté que la taille des grains est de l'ordre de 150µm.

### Exemple 4 : Obtention de l'hexanitrohexaazaisowurtzitane de forme polymorphe epsilon. Méthode par ensemencement en milieu acétone/toluène.

On mélange, à la température ambiante proche de 20°C, 10 g d'hexanitrohexaazaisowurtzitane obtenu selon l'exemple 2 avec 20 ml d'acétone. On obtient ainsi une solution saturée dans laquelle une faible partie de l'hexanitrohexaazaisowurtzitane de départ reste en suspension.

On ajoute 80 ml de toluène, ce qui permet d'obtenir une solution saturée d'hexanitrohexaazaisowurtzitane dans un mélange 20/80 en volumes acétone/toluène respectivement dans laquelle une faible partie de l'hexanitrohexaazaisowurtzitane de départ reste en suspension.

On filtre cette suspension et recueille le filtrat, c'est à dire la solution saturée, que l'on ensemence ensuite par quelques cristaux d'hexanitrohexaazaisowurtzitane de forme polymorphe epsilon obtenu selon l'exemple 3.

On concentre ensuite le mélange à 25°C sous une pression partielle de 2,5 10³-5 10³Pa (environ 20-40 mm Hg) jusqu'à ce que tout l'acétone soit éliminé.

Un solide blanc (9 g) précipite au cours de cette opération de concentration, que l'on récupère par filtration.

Ce solide blanc, identifié et analysé comme décrit pour l'exemple 3, qui présente toutes les caractéristiques, notamment physiques et spectrales, du produit obtenu pour l'exemple 3, est de l'hexanitrohexaazaisowurtzitane de forme polymorphe epsilon présentant un diamètre médian de 150µm environ.

Cet exemple a été reproduit d'une part en utilisant des semences de diverses granulométries comprises entre 15µm et 80µm, obtenues par broyage aux ultra-sons de la semence utilisée, et d'autre part en faisant varier la vitesse d'évaporation de l'acétone.

La caractérisation de la morphologie des grains par microscopie optique montre que les grains obtenus sont toujours des agglomérats de diamètre supérieur à 100µm et que la granulométrie de la semence a peu d'influence sur la granulométrie finale.

### Exemples 5 à 16 : Obtention, selon l'invention, de diverses coupes granulométriques d'hexanitrohexaazaisowurtzitane de forme polymorphe epsilon, en milieu acétate d'éthyle/toluène.

Les exemples à l'échelle laboratoire (20 g ou 30 g environ) ont été réalisés dans un réacteur double enveloppe en verre de laboratoire d'un volume de 250 ml ou 500 ml selon les exemples, équipé d'une agitation mécanique par pales, d'une sonde de température, d'une colonne à distiller avec un système de régulation du taux de reflux surmontée d'un réfrigérant à - 20°C et d'un piège à carboglace, et d'une recette pour recueillir les condensats.

Les exemples à l'échelle pilote (3,5 kg environ) ont été réalisés dans un réacteur en inox double enveloppe de 60 l comportant le même type d'équipement.

### Exemples 5 à 11 : Obtention d'une coupe granulométrique ayant un diamètre médian compris entre 20µm et 40µm.

### Exemple 5 :

On dissout, à la température ambiante proche de 20°C, 30 g d'hexanitrohexaazaisowurtzitane de forme polymorphe alpha obtenu selon l'exemple 2 dans 90 ml d'acétate d'éthyle, puis on ajoute 210 ml de toluène. On obtient ainsi une solution saturée en hexanitrohexaazaisowurtzitane dans un mélange acétate d'éthyle/toluène 30/70 respectivement en volumes.

On ensemence ensuite cette solution avec 0,6 g d'hexanitrohexaazaisowurtzitane (2% par rapport au produit de départ) de forme polymorphe epsilon obtenu selon l'exemple 3, préalablement broyé aux ultrasons et présentant un diamètre médian compris entre 5µm et 10µm (détermination par microscopie optique), puis on concentre la solution par évaporation de l'acétate d'éthyle, sous agitation de 150 tours/min, sous pression partielle de 6,25 10³ Pa (environ 50 mmHg) assurée au moyen d'une pompe à anneau liquide et d'une vanne de régulation, et à la température ambiante d'environ 20°C.

Le taux de reflux est ajusté de façon à distiller environ 90% de l'acétate d'éthyle initial en 3 h environ.

Un solide blanc (27,5 g) précipite au cours de cette opération de concentration, que l'on récupère par filtration (rendement 90%).

Ce solide blanc, identifié et analysé comme décrit pour les exemples 3 et 4, qui présente toutes les caractéristiques, notamment physiques et spectrales, du produit obtenu pour ces exemples 3 et 4, est de l'hexanitrohexaazaisowurtzitane de forme polymorphe epsilon présentant un diamètre médian compris entre 20µm et 40µm (détermination par microscopie optique).

Les grains présentent des faces lisses et un faciès régulier (observation par microscopie optique).

### Exemples 6 à 9

On a reproduit l'exemple 5, à l'échelle pilote, à partir de 3,5 kg d'hexanitrohexaazaisowurtzitane de forme polymorphe alpha obtenu selon l'exemple 2.

Les variations de conditions expérimentales et les résultats obtenus sont les suivants :

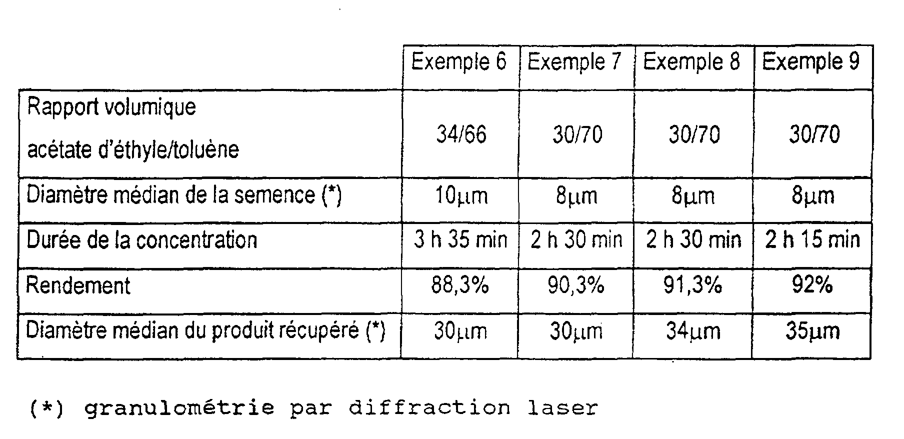

### Exemples 10 et 11 :

On a reproduit l'exemple 5, mais avec 0,3 g (1%) de semence pour l'exemple 10 et 1,2 g (4%) pour l'exemple 11.

On obtient les mêmes résultats que pour l'exemple 5.

### Exemples 12 à 15 : Obtention d'une coupe granulométrique ayant un diamètre médian compris entre 40µm et 60µm.

### Exemple 12 :

On mélange, à la température ambiante d'environ 20°C, 30 g d'hexanitrohexaazaisowurtzitane de forme polymorphe alpha obtenu selon l'exemple 2 avec un mélange de 120 ml d'acétate d'éthyle et de 180 ml de toluène.

On obtient une solution saturée dans laquelle une faible partie de l'hexanitrohexaazaisowurtzitane de départ reste en suspension. On filtre cette suspension et recueille le filtrat, c'est à dire la solution saturée, que l'on ensemence ensuite par 0,6 g d'hexanitrohexaazaisowurtzitane de forme polymorphe epsilon obtenu selon l'exemple 4, préalablement broyé aux ultrasons et présentant un diamètre médian compris entre 10µm et 15µm.

On concentre la solution par évaporation de l'acétate d'éthyle dans les mêmes conditions que pour l'exemple 5, mais en arrêtant la distillation après avoir évaporé 80% de l'acétate d'éthyle de départ (durée 3 h environ).

On récupère 26 g (rendement 85%) d'hexanitrohexaazaisowurtzitane de forme polymorphe epsilon présentant d'une part un diamètre médian compris entre 40µm et 60µm et d'autre part des faces lisses et un faciès régulier.

### Exemples 13 et 14 :

On a reproduit l'exemple 12, mais en concentrant la solution à une température de 40°C pour l'exemple 13 et de 75°C pour l'exemple 14.

Pour l'exemple 13, on obtient les mêmes résultats que pour l'exemple 12.

Pour l'exemple 14, le produit obtenu est un mélange des formes polymorphes epsilon et gamma de l'hexanitrohexaazaisowurtzitane et se présente sous forme d'aiguilles et d'agglomérats.

### Exemple 15 :

On a reproduit l'exemple 12, mais à partir de 30 g d'hexanitrohexaazaisowurtzitane de forme polymorphe epsilon (au lieu de la forme alpha) obtenu selon l'exemple 4.

On obtient les mêmes résultats que pour l'exemple 12.

### Exemple 16 : Obtention d'une coupe granulométrique ayant un diamètre médian compris entre 80µm et 100µm.

On a reproduit l'exemple 12, mais avec une semence présentant un diamètre médian compris entre 25µm et 40µm.

L'hexanitrohexaazaisowurtzitane de forme polymorphe epsilon récupéré (rendement 87%) présente un diamètre médian compris entre 80µm et 100µm.

### Exemples 17 à 23 : Obtention, selon l'invention, de diverses coupes granulométriques comprises dans la gamme 5µm-100µm d'hexanitrohexaazaisowurtzitane de forme polymorphe epsilon, en divers milieux solvant/non solvant.

On utilise les mêmes appareillages que pour les exemples 5 à 16.

### Exemple 17 : En milieu acétate d'isopropyle/toluène 30/70 en volumes respectivement.

On mélange, à la température ambiante de 20°C, 20 g d'hexanitrohexaazaisowurtzitane de forme polymorphe alpha obtenu selon l'exemple 2 avec un mélange de 60 ml d'acétate d'isopropyle et 140 ml de toluène. On obtient une solution saturée dans laquelle une faible partie de l'hexanitrohexaazaisowurtzitane de départ reste en suspension. On filtre cette suspension et recueille le filtrat que l'on ensemence par 0,4 g (2%) d'hexanitrohexaazaisowurtzitane de forme polymorphe epsilon obtenu selon l'exemple 4, préalablement broyé aux ultrasons et présentant un diamètre médian de 5µm environ.

On concentre la solution par évaporation de l'acétate d'isopropyle, dans les mêmes conditions expérimentales que pour l'exemple 5.

On récupère, avec un rendement de 92%, de l'hexanitrohexaazaisowurtzitane de forme polymorphe epsilon présentant d'une part un diamètre médian compris entre 5µm et 10µm et d'autre part des grains non agglomérées de forme régulière.

### Exemple 18 : En milieu acétate de méthyle/toluène 30/70 en volumes respectivement.

On a reproduit l'exemple 17 en utilisant l'acétate de méthyle au lieu de l'acétate d'isopropyle et une semence de diamètre médian 13µm au lieu de 5µm.

L'hexanitrohexaazaisowurtzitane de forme polymorphe epsilon, récupéré avec un rendement de 92%, présente un diamètre médian de 10µm à 15µm.

### Exemple 19 : En milieu acétate d'éthyle/1,2-dichloroéthane 30/70 en volumes respectivement.

On a reproduit l'exemple 17 en utilisant l'acétate d'éthyle au lieu de l'acétate d'isopropyle et le 1,2-dichloroéthane au lieu du toluène.

L'hexanitrohexaazaisowurtzitane de forme polymorphe epsilon récupéré (rendement 51%) se présente sous forme d'amas ayant un diamètre médian de 15µm environ.

### Exemple 20 : En milieu acétate d'éthyle/xylène 30/70 en volumes respectivement.

On a reproduit l'exemple 19 en utilisant l'ortho xylène au lieu du 1,2-dichloroéthane.

L'hexanitrohexaazaisowurtzitane de forme polymorphe epsilon récupéré (rendement 92%) se présente sous forme de grains réguliers ayant un diamètre médian de 20µm à 25µm.

### Exemple 21 : En milieu THF/toluène 22/78 en volumes respectivement.

On a reproduit l'exemple 17 en utilisant un mélange de 44 ml de THF et de 156 ml de toluène.

L'hexanitrohexaazaisowurtzitane de forme polymorphe epsilon récupéré (rendement 92%) se présente sous forme d'agglomérats ayant un diamètre médian de 60µm à 70µm.

### Exemple 22 : En milieu acétonitrile/acétate d'éthyle/toluène 21/5/74 en volumes respectivement.

On a reproduit l'exemple 17 en utilisant un mélange de 42 ml d'acétonitrile, 10 ml d'acétate d'éthyle et 148 ml de toluène.

On récupère, avec un rendement de 77%, de l'hexanitrohexaazaisowurtzitane de forme polymorphe epsilon se présentant sous forme d'agglomérats ayant un diamètre médian de 80µm environ.

### Exemple 23 : En milieu méthyléthylcétone/toluène 20/80 en volumes respectivement.

On a reproduit l'exemple 17 en utilisant un mélange de 40 ml de méthyléthylcétone et de 160 ml de toluène.

On récupère, avec un rendement de 92%, de l'hexanitrohexaazaisowurtzitane de forme polymorphe epsilon se présentant sous forme d'agglomérats ayant un diamètre médian de 80µm environ.

## Revendications

1. Procédé d'obtention de la forme polymorphe epsilon de l'hexanitrohexaazaisowurtzitane, **caractérisé en ce que** :
- on réalise une solution saturée d'hexanitrohexaazaisowurtzitane de forme polymorphe quelconque dans un mélange comprenant d'une part un solvant organique de l'hexanitrohexaazaisowurtzitane choisi dans le groupe constitué par les esters, les nitriles, les éthers, les cétones à l'exclusion de l'acétone, et leurs mélanges, et d'autre part un non solvant de l'hexanitrohexaazaisowurtzitane choisi dans le groupe constitué par les hydrocarbures aliphatiques, les hydrocarbures aromatiques et leurs mélanges, le solvant de l'hexanitrohexaazaisowurtzitane étant plus volatil que le non solvant,
- on ensemence cette solution saturée par quelques cristaux d'hexanitrohexaazaisowurtzitane de forme polymorphe epsilon,
- on concentre ensuite la solution par évaporation du solvant.

2. Procédé selon la revendication 1, **caractérisé en ce que** le mélange comprend un solvant organique choisi dans le groupe constitué par les esters et un non solvant choisi dans le groupe constitué par les alcanes, les hydrocarbures aliphatiques halogénés et les hydrocarbures aromatiques.

3. Procédé selon la revendication 2, **caractérisé en ce que** le mélange comprend un solvant organique choisi dans le groupe constitué par les formiates et les acétates et un non solvant choisi dans le groupe constitué par les hydrocarbures aromatiques.

4. Procédé selon la revendication 3, **caractérisé en ce que** le solvant organique est un acétate choisi dans le groupe constitué par l'acétate de méthyle, l'acétate d'éthyle et l'acétate d'isopropyle, et **en ce que** le non solvant est un hydrocarbure aromatique choisi dans le groupe constitué par le toluène et les xylènes.

5. Procédé selon la revendication 4, **caractérisé en ce que** le solvant organique est l'acétate d'éthyle et **en ce que** le non solvant est le toluène.

6. Procédé selon la revendication 1, **caractérisé en ce que** le rapport volumique solvant/non solvant respectivement, est compris entre 10/90 et 50/50.

7. Procédé selon la revendication 1, **caractérisé en ce que** la quantité de semence représente entre 0,2% et 5% en poids de la quantité d'hexanitrohexaazaisowurtzitane de forme polymorphe quelconque.

8. Procédé selon la revendication 1, **caractérisé en ce que** lors de l'opération de concentration de la solution par évaporation du solvant, la température n'excède pas 50°C.

9. Procédé selon la revendication 1, **caractérisé en ce qu'**on réalise d'abord une solution d'hexanitrohexaazaisowurtzitane de forme polymorphe quelconque dans le solvant organique ou dans le solvant organique et une partie du non solvant et **en ce qu'**on ajoute ensuite le non solvant ou le reste du non solvant.

## Patentansprüche

1. Verfahren zur Herstellung der ε-polymorphen Form von Hexanitrohexaazaisowurtzitan, das durch folgende Schritte gekennzeichnet ist:
- Herstellen einer gesättigten Lösung eines in einer beliebigen polymorphen Form vorliegenden Hexanitrohexaazaisowurtzitans in einem Gemisch, das ein organisches Lösemittel für Hexanitrohexaazaisowurtzitan, das unter den Estern, Nitrilen, Ethern, Ketonen, wobei Aceton ausgeschlossen ist, und den Gemischen dieser Lösemittel ausgewählt ist, und ein Nichtlösemittel von Hexanitrohexaazaisowurtzitan, das unter den aliphatischen Kohlenwasserstoffen, aromatischen Kohlenwasserstoffen und den Gemischen dieser Kohlenwasserstoffe ausgewählt ist, enthält, wobei das Lösemittel für Hexanitrohexaazaisowurtzitan leichter flüchtig als das Nichtlösemittel ist,
- Beimpfen dieser gesättigten Lösung mit einigen Kristallen Hexanitrohexaazaisowurtzitan mit der ε-polymorphen Form,
- Aufkonzentrieren der Lösung durch Verdampfen des Lösemittels.

2. Verfahren nach Anspruch 1, **dadurch gekennzeichnet, daß** das Gemisch ein organisches Lösemittel, das unter den Estern ausgewählt ist, und ein Nichtlösemittel, das unter den Alkanen, halogenhaltigen aliphatischen Kohlenwasserstoffen und aromatischen Kohlenwasserstoffen ausgewählt ist, enthält.

3. Verfahren nach Anspruch 2, **dadurch gekennzeichnet, daß** das Gemisch ein organisches Lösemittel, das unter den Formiaten und Acetaten ausgewählt ist, und ein Nichtlösemittel, das unter den aromatischen Kohlenwasserstoffen ausgewählt ist, enthält.

4. Verfahren nach Anspruch 3, **dadurch gekennzeichnet, daß** das organische Lösemittel ein Acetat ist, das unter Methylacetat, Ethylacetat und Isopropylacetat ausgewählt ist, und daß das Nichtlösemittel ein aromatischer Kohlenwasserstoff ist, der unter Toluol und den Xylolen ausgewählt ist.

5. Verfahren nach Anspruch 4, **dadurch gekennzeichnet, daß** es sich bei dem organischen Lösemittel um Ethylacetat und dem Nichtlösemittel um Toluol handelt.

6. Verfahren nach Anspruch 1, **dadurch gekennzeichnet, daß** das Volumenverhältnis Lösemittel/Nichtlösemittel im Bereich von 10/90 bis 50/50 liegt.

7. Verfahren nach Anspruch 1, **dadurch gekennzeichnet, daß** die Menge an Impfkristallen im Bereich von 0,2 bis 5 Gew.-%, bezogen auf die Menge an Hexanitrohexaazaisowurtzitan, das in einer beliebigen polymorphen Form vorliegt, liegt.

8. Verfahren nach Anspruch 1, **dadurch gekennzeichnet, daß** die Temperatur beim Aufkonzentrieren der Lösung durch Verdampfen des Lösemittels 50 °C nicht übersteigt.

9. Verfahren nach Anspruch 1, **dadurch gekennzeichnet, daß** zunächst eine Lösung des in einer beliebigen polymorphen Form vorliegenden Hexanitrohexaazaisowurtzitans in dem organischen Lösemittel oder dem organischen Lösemittel und einem Teil des Nichtlösemittels hergestellt wird und daß anschließend das Nichtlösemittel oder das restliche Nichtlösemittel zugegeben wird.

## Claims

1. Method for obtaining the epsilon polymorphic form of hexanitrohexaazaisowurtzitane, **characterized in that**:
- a saturated solution is prepared of hexanitrohexaazaisowurtzitane in any polymorphic form in a mixture comprising on the one hand an organic solvent for hexanitrohexaazaisowurtzitane chosen from the group consisting of esters, nitriles, ethers, ketones excluding acetone, and mixtures thereof and, on the other hand, a non-solvent for hexanitrohexaazaisowurtzitane chosen from the group consisting of aliphatic hydrocarbons, aromatic hydrocarbons and mixtures thereof, the solvent for hexanitrohexaazaisowurtzitane being more volatile than the non-solvent,
- the saturated solution is seeded with a few crystals of hexanitrohexaazaisowurtzitane in the epsilon polymorphic form,
- the solution is then concentrated by evaporating the solvent.

2. Method according to claim 1, **characterized in that** the mixture comprises an organic solvent chosen from the group consisting of esters and a non-solvent chosen from the group consisting of alkanes, halogenated aliphatic hydrocarbons and aromatic hydrocarbons.

3. Method according to claim 1, **characterized in that** the mixture comprises an organic solvent chosen from the group consisting of formates and acetates and a non-solvent chosen from the group consisting of aromatic hydrocarbons.

4. Method according to claim 3, **characterized in that** the organic solvent is an acetate chosen from the group consisting of methyl acetate, ethyl acetate and isopropyl acetate, and **in that** the non-solvent is an aromatic hydrocarbon chosen from the group consisting of toluene and xylenes.

5. Method according to claim 4, **characterized in that** the organic solvent is ethyl acetate and the non-solvent is toluene.

6. Method according to claim 1, **characterized in that** the ratio by volume of solvent/non-solvent respectively, lies between 10/90 and 50/50.

7. Method according to claim 1, **characterized in that** the quantity of seed represents between 0.2 % and 5 % by weight of the quantity of hexanitrohexaazaisowurtzitane of any polymorphic form.

8. Method according to claim 1, **characterized in that** during the operation of concentrating the solution by evaporating the solvent, the temperature does not exceed 50°C.

9. Method according to claim 1, **characterized in that** a solution of hexanitrohexaazaisowurtzitane of any polymorphic form is first of all prepared in the organic solvent or in the organic solvent and part of the non-solvent and **in that** the non-solvent or the remainder of the non-solvent is then added.
